# EUROPÄISCHE PATENTANMELDUNG

(11) **EP 0 976 387 A1**
(43) Veröffentlichungstag der Anmeldung: **02.02.2000**
(21) Anmeldenummer: 99113969.2
(22) Anmeldetag: 17.07.1999
(51) Int. Cl.: A61K 7/38

(54) **Verfahren zur Herstellung von stabilen Antiperspirant-Zusammensetzungen als wässrige Lösung und als Pulver**

(30) Priorität: 20.07.1998 DE 19832485
(71) Anmelder: BK Giulini Chemie GmbH & Co. OHG, 67065 Ludwigshafen (DE)
(72) Erfinder: Kaufmann, Bruno, 67227 Frankenthal (DE); Reibel, Wolfgang, Dr., 67063 Ludwigshafen (DE); Schanz, dr.Klaus, 67125 Dannstadt-Schauernheim (DE); Mürtz, Helmut, Dr., 67122 Altrip (DE)

(57) **Zusammenfassung**

Die Erfindung betrifft ein Verfahren zur Herstellung einer Antiperspirant-Zusammensetzung, die nach dem Verfahren hergestellte Antiperspirant-Zusammensetzung selbst sowie deren erfindungsgemäße Verwendung. Das Verfahren ist charakterisiert durch die Zugabe von einer Phosphorsäure. Die Zugabe der Phosphorsäure hat einen großen technologischen Vorteil, ein Gelieren der Lösungen wird hierdurch verhindert.

## Beschreibung

Die Erfindung betrifft ein Verfahren zur Herstellung einer Antiperspirant-Zusammensetzung, die nach dem Verfahren hergestellte Antiperspirant-Zusammensetzung selbst sowie deren erfindungsgemäße Verwendung.

Antiperspirant-Zusammensetzungen auf der Basis eines Aluminium- und eines Zirkoniumhydroxids zur Verhinderung oder zumindest Verminderung von Schweißbildung, beispielsweise unter den Achseln, sind im Stand der Technik bekannt. Dabei werden in kosmetischen Zubereitungen bevorzugt Aluminium- und Zirkoniumhydroxychlorid eingesetzt.

Die Herstellung solcher Antiperspirant-Zusammensetzungen bereitet in der Praxis allerdings erhebliche Probleme, weil die resultierenden Lösungen nach einem mehr oder weniger kurzen Zeitraum zum Gelieren neigen. Damit ist meistens eine Trübung verbunden, wodurch die Weiterverarbeitung zu kosmetischen Zubereitungen, z. B. in Form von Roll on-Stiften oder die Herstellung eines Pulvers unmöglich gemacht werden.

Es ist daher im Stand der Technik auch bereits bekannt, den Antiperspirant-Zusammensetzungen weitere Zusätze hinzuzufügen und/oder eine thermische Behandlung vorzusehen. Damit soll zum einen das unerwünschte Gelieren verhindert und zum anderen eine Steigerung der schweißhemmenden Wirkung der Zusammensetzungen erreicht werden.

So offenbart US-A-2,814,584 eine Antiperspirant-Zusammensetzung auf der Basis einer Hafnium- und/oder Zirkoniumverbindung und eines Aluminiumhydroxids, die als weitere Zusätze eine anorganische Säure und Harnstoff enthält. Dabei soll der Harnstoff das Gelieren verhindern.

US-A 4, 331, 609 offenbart eine Antiperspirant-Zusammensetzung, die neben einem Aluminium- und Zirkoniumhydroxid eine wasserlösliche neutrale Aminosäure, wie Glycin, und eine anorganische Säure in bestimmten Molverhältnissen aufweist.

In EP-A-0 393 275 wird ein Aluminium-Zirkonium-Hydroxyhalogenid-Glycin-Komplex offenbart, bei dem die Ausgangsverbindungen in Form eines Aluminium- und Zirkoniumhydroxyhalogenids zur Steigerung der schweißhemmenden Wirkung und zur Erzielung stabiler Lösungen bei erhöhter Temperatur umgesetzt werden. Durch die Notwendigkeit einer Temperaturerhöhung erhöhen sich aber gleichzeitig die Betriebskosten, wodurch das Verfahren unwirtschaftlicher wird. Außerdem wird es durch die Beobachtung der Temperaturführung aufwendiger.

In EP-B-0 337 464 wird Aluminiumhydroxychlorid mit ortho-Kieselsäure umgesetzt. Auch hier kann eine Temperaturerhöhung vorgesehen sein. Zusätzlich kann Zirkoniumhydroxychlorid zugesetzt werden.

Ausgehend von diesem Stand der Technik lag der vorliegenden Erfindung die Aufgabe zugrunde, ein Verfahren zur Herstellung einer Antiperspirant-Zusammensetzung bereitzustellen, bei dem klare und lagerstabile Lösungen mit hoher schweißhemmender Wirkung erhalten werden.

Gelöst wird diese Aufgabe durch ein Verfahren, bei dem eine Aluminiumverbindung der allgemeinen Formel

Al₂ (OH)₆₋ₙ Xₙ,

mit X = Cl⁻, Br⁻, J⁻, NH₂SO₃⁻, SO₄², NO₃⁻ oder Mischungen von zwei oder mehreren dieser Anionen, und n = 0,5 - 6,
mit einer Phosphorsäure, einer Zirkoniumverbindung der allgemeinen Formel

   Zr O (OH)₂₋ₚYₚ,
mit Y = Cl⁻, Br⁻, J⁻ oder Mischungen von zwei oder mehreren dieser Anionen, und p = 0,5- 2,
und gegebenenfalls einer wasserlöslichen neutralen Aminosäure umgesetzt wird.

Durch den Zusatz von Phosphorsäure werden klare und viskositätsstabile Antiperspirant-Zusammensetzungen erhalten, die auch nach längerer Lagerung nicht gelieren. Bei den Aluminium- und Zirkoniumhydroxy-Verbindungen wurden bevorzugt die jeweiligen Chloride verwendet.

Gemäß einer bevorzugten Ausführungsform kann die Aluminiumverbindung zunächst mit der Phosphorsäure versetzt werden. Die Verwendung von ortho-Phosphorsäure, vorzugsweise eine 5 - 75 %igen ortho-Phosphorsäure, besonders bevorzugt mit einer Konzentration von 25 - 55 Gew.%, hat sich bei der Durchführung des erfindungsgemäßen Verfahrens als besonders günstig erwiesen.

Ebenso wurden besonders günstige Ergebnisse erzielt, wenn das Verhältnis von Al : P₂O₅ 10 - 140, vorzugsweise 17 - 60 beträgt und als Aminosäure Glycin verwendet wird, das Verhältnis von Zr: Glycin 0,5-2, bevorzugt 0,8-1,2 und von (Al+Zr):Cl 0,9-2,1, beträgt.

Bei der Herstellung der Antiperspirant-Zusammensetzung wird zunächst eine Lösung erhalten, die dann, wenn ein Pulver hergestellt werden soll, in an sich bekannter Weise getrocknet werden kann. Als solche Trocknungsverfahren kommen die Vakuumtrocknung und die Sprühtrocknung in Frage, wovon die Sprühtrocknung bevorzugt ist.

Die Erfindung betrifft auch eine Antiperspirant-Zusammensetzung, die nach dem oben angegebenen Verfahren erhalten wird. Diese Antiperspirant-Zusammensetzung kann beispielsweise in Form einer wäßrigen, alkoholischen Lösung vorliegen.

Die Erfindung betrifft des weiteren eine kosmetische Zubereitung, die die zuvor beschriebene Antiperspirant-Zusammensetzung enthält.

Im folgenden soll die Erfindung anhand von Beispielen näher erläutert werden.

### Beispiel 1: Glycin-freie Antiperspirant-Lösung

In einem 250 ml Becherglas werden 157,6 g einer Aluminiumhydroxychlorid-Lösung der allgemeinen Formel Al₂(OH)₅Cl mit einem Gehalt von 23,2 % Al₂O₃ bei Raumtemperatur vorgelegt. Unter Rühren gibt man 0,02 Mol einer 75%igen ortho-Phosphorsäure-Lösung (im folgenden als H₃PO₄-Lösung bezeichnet) langsam zu. Anschließend wird zu dieser klaren Lösung 36,4 g einer Zirkoniumoxychlorid-Lösung der allgemeinen Formel ZrOCl₂ mit 34,5 % ZrO₂ zugetropft. Die so erhaltene Lösung ist dünnflüssig, klar und auch nach 3 Monaten im Schaukeltest (+5°C/+45°C) viskositätsstabil.

Die Gehalte der Lösung sind:19,8% Al₂O₃, 6,21 % ZrO₂ und 8,8 % Cl.

### Vergleichsbeispiei 1:

Es wurde verfahren, wie bei Beispiel 1 angegeben, jedoch keine H₃PO₄-Lösung hinzugefügt. Nach Beendigung der Umsetzung erfolgte eine spontane Gelbildung. Eine Weiterverarbeitung war nicht mehr möglich.

### Beispiel 2: Glycin-freies Antiperspirant-Pulver

In einem 100 1 Reaktor wird eine Aluminiumhydroxychlorid-Lösung mit einem Gehalt von 10,2 % Aluminium und 8,7 % Chlorid bei 30°C vorgelegt. Zu 81,9 kg dieser Lösung gibt man unter Rühren 3 kg einer 75%igen H₃PO₄-Lösung. Danach erfolgt die Zugabe einer Zirkoniumoxychlorid-Lösung mit 25,3 % Zirkonium und 9,6 % Chlorid. Die so hergestellte Lösung enthält: 8,3 % Al, 3,8 % Zr und 8,6 % Cl.

Ein Teil dieser Lösung wurde in einem Sprühtrockner getrocknet. Das so hergestellte Pulver enthält: 21,4% Al, 9,8% Zr, 18,9% Cl.

### Beispiel 3:

In einem 2,5 1 Becherglas wird eine Aluminiumhydroxychlorid-Lösung mit einem Gehalt von 10,5 % Al und 9,6 % Chlorid vorgelegt. Zu 1510 g dieser Lösung gibt man unter Rühren 40 g einer 75 %igen H₃PO₄-Lösung. Danach erfolgt die Zugabe einer Zirkoniumoxychlorid-Lösung mit 25,5 % Zr und 10,4 % Cl. Die so erhaltene Lösung enthält 10,1 % Al, 5,0 % Zr und 9,3 % Cl.

### Beispiele 4-7: Glycin-haltige Antiperspirant-Lösung

In einem 250 ml Becherglas wird eine Aluminiumhydroxychlorid-Lösung mit einem Al : Cl-Verhältnis von 1,86 und den in Tabelle 1 angegebenen Mengen vorgelegt. Bei einer Temperatur von 20°C gibt man unter Rühren 4 g einer 75%igen H₃PO₄-Lösung zu. In einem zweiten Beeherglas wird in einer Zirkoniumoxychlorid-Lösung der allgemeinen Formel ZrO(OH)Cl mit einem Gehalt von 34,5 % ZrO₂ und 10,4 % Cl Glycin gelöst. Die so erhaltene klare Zr-Glycin-Lösung wird zu der ersten Lösung unter Rühren zugetropft.
Es entstehen klare, dünnflüssige Lösungen der folgenden Zusammensetzung. Die Gehalte sind in Tabelle 1 zusammengefaßt.

**Tabelle 1**

| | 4 | 5 | 6 | 7 |
|---|---|---|---|---|
| g Al-Lsg. | 141,7 | 158,0 | 120,5 | 110,8 |
| g Zr-Lösung | 37,5 | 31,4 | 54,8 | 70,5 |
| g Glycin | 7,9 | 6,6 | 11,5 | 14,8 |
| g HCl 32% | 8,9 | 0 | 9,2 | 0 |
| g H₃PO₄ 75% | 4,0 | 4,0 | 4,0 | 4,0 |

| Gehalte: | | | | |
|---|---|---|---|---|
| % Al | 8,5 | 9,5 | 7.2 | 6,7 |
| % Zr | 4,8 | 4,0 | 7,0 | 9,0 |
| %Cl | 9,4 | 8,3 | 9,4 | 8,4 |
| % Glycin | 3,9 | 3,3 | 5,8 | 7,4 |

## Patentansprüche

1. Verfahren zur Herstellung einer Antiperspirant-Zusammensetzung, bei dem eine Aluminiumverbindung der allgemeinen Formel
Al₂ (OH)₆₋ₙ X ₙ,
mit X = Cl⁻, Br⁻, J⁻, NH₂SO₃⁻, SO₄², NO₃⁻ oder Mischungen von zwei oder mehreren dieser Anionen, und n = 0,5 - 6,
mit einer Phosphorsäure, einer Zirkoniumverbindung der allgemeinen Formel
Zr O (OH)₂₋ₚ Yₚ,
mit Y = Cl⁻, Br⁻, J⁻ oder Mischungen von zwei oder mehreren dieser Anionen, und p= 1 - 2
und gegebenenfalls einer wasserlöslichen neutralen Aminosäure umgesetzt wird.

2. Verfahren nach Anspruch 1, dadurch gekennzeichnet, daß die Aluminiumverbindung zunächst mit der Phosphorsäure versetzt wird.

3. Verfahren nach Anspruch 1 oder 2, dadurch gekennzeichnet, daß ortho-Phosphorsäure eingesetzt wird.

4. Verfahren nach einem der Ansprüche 1 bis 3, dadurch gekennzeichnet, daß eine 5 - 75 %ige ortho-Phosphorsäure eingesetzt wird.

5. Verfahren nach einem der Ansprüche 1 bis 4, dadurch gekennzeichnet, daß das Verhältnis Al : P₂O₅ 10 - 140, vorzugsweise 17 - 60 beträgt.

6. Verfahren nach einem der Ansprüche 1 bis 5, dadurch gekennzeichnet, daß als Aminosäure Glycin verwendet wird.

7. Verfahren nach einem der Ansprüche 1 bis 6, dadurch gekennzeichnet, daß die Zusammensetzung bei der Umsetzung als Lösung erhalten wird oder in an sich bekannter Weise getrocknet wird.

8. Antiperspirant-Zusammensetzung erhältlich nach dem Verfahren nach einer der Ansprüche 1 bis 7.

9. Antiperspirant-Zusammensetzung nach Anspruch 8 in Form einer wäßrigen, alkoholischen Lösung.

10. Kosmetische Zubereitung, die eine Antiperspirant-Zusammensetzung gemäß Anspruch 8 oder 9 erhält.
